# FASCICULE DE BREVET EUROPEEN

(11) **EP 0 955 310 B1**
(45) Date de publication et mention de la délivrance du brevet: **13.10.2004**
(21) Numéro de dépôt: 99401019.7
(22) Date de dépôt: 26.04.1999
(51) Int. Cl.: C07K 5/06

(54) **Nouveaux dérivés de l'acide (3,4,7,8,9,10) -hexahydro-6,10-dioxo-6H-pyridazino /1,2-a/ /1,2/ diazépine-1-carboxylique, leur procédé de préparation et leur application à la préparation de médicaments**
Neue (3,4,7,8,9,10) hexahydro-6, 10-dioxo-6H-pyridazino /1, 2-a/ /1, 2/ Diazepin-1-Carbonsäure-Derivate, Verfahren zu deren Herstellung und deren Verwendung zur Herstellung pharmazeutischer Wirkstoffe
New derivatives of (3,4,7,8,9,10)-hexahydro-6,10-dioxo-6H-pyridazino /1,2-a/ /1,2/ diazepine-1-carboxylic acid, process for their preparation and use for preparation of pharmaceutical agents

(30) Priorité: 27.04.1998 FR 9805242
(43) Date de publication de la demande: 10.11.1999
(73) Titulaire: Aventis Pharma S.A., 92160 Antony (FR)
(72) Inventeur: Brion, Francis, 31130 Balma (FR); Crocq, Véronique, 21000 Dijon (FR); Roussel, Patrick, 94320 Thiais (FR)

(56) Documents cités:
- EP-A- 0 570 764
- WO-A-97/22619
- US-A- 4 512 924
- US-A- 5 723 602

## Description

La présente invention concerne des dérivés de l'acide (3,4,7,8,9,10-hexahydro-6,10-dioxo-6H-pyridazino[1,2-a] [1,2] diazépine-1-carboxylique, leur procédé de préparation et leur application à la préparation de composés thérapeutiquement actifs.

Les brevets US 4,512,924, US 5,723,602 ainsi que la demande WO 97/22619 décrivent des dérivés de pyridazodiazépines pour leur urilisation à titre d'inhibiteurs d'ACE ou d'ICE ainsi que des intermédiaires de synthèse de formule (III).

L'invention a pour objet à titre de produits chimiques nouveaux, les composés de formule (I) : 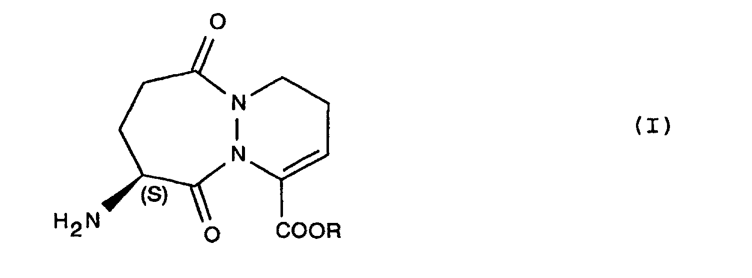 dans lesquels R représente un atome d'hydrogène, un radical alkyle ou aralkyle renfermant jusqu'à 18 atomes de carbone, la fonction amine pouvant être libre ou protégée.
R représente par exemple un radical méthyle, éthyle, propyle, isopropyle, n-butyle, isobutyle ou tertbutyle, ou un radical benzyle ou naphthyle. Lorsque la fonction amine est protégée, la protection peut se faire selon les méthodes classiques de protection des amines, et l'invention a notamment pour objet les composés de formule (I), dans lesquels la fonction amine est protégée, qui répondent à la formule (IA) : 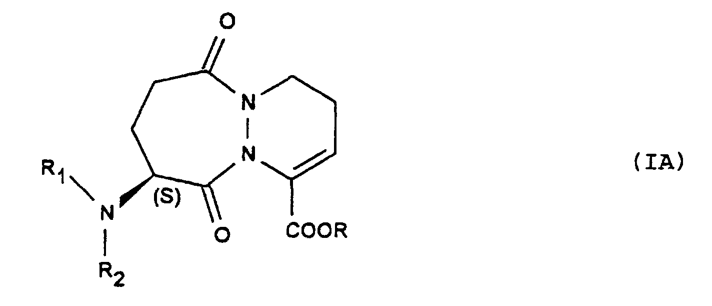 dans laquelle R conserve sa signification précédente et/ou bien R₁ représente un radical : 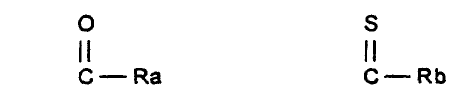 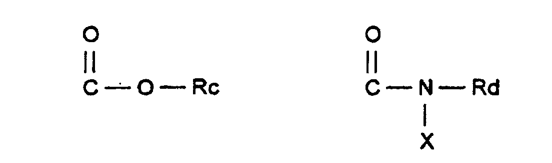
Ra, Rb, Rc et Rd représentant un radical alkyle ou aryle renfermant jusqu'à 18 atomes de carbone, ou un radical mono ou polycyclique renfermant un ou plusieurs hétéroatomes,
X représentant un atome d'hydrogène, un radical alkyle renfermant jusqu'à 8 atomes de carbone ou un radical aryle renfermant jusqu'à 14 atomes de carbone,
et R₂ représente un atome d'hydrogène,
ou bien R₁ et R₂ forment ensemble un radical mono ou polycyclique renfermant un ou plusieurs hétéroatomes.

On peut utiliser pour protéger les amines, les composés cycliques par exemple le radical : 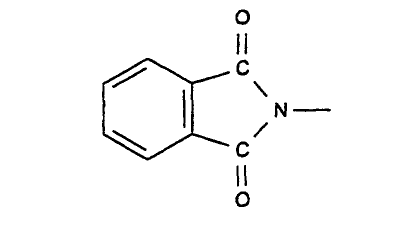 ou encore le radical :

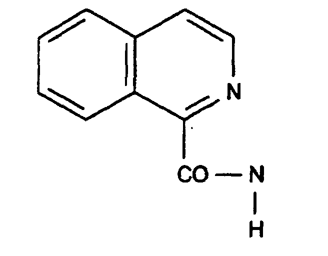

L' invention a plus particulièrement pour objet les composés de formule (I) dans lesquels R₁ et R₂ forment ensemble un radical polycyclique renfermant un ou plusieurs hétéroatomes.

Parmi les composés préférés de l'invention, on peut citer les composés de formule (IA₁) : 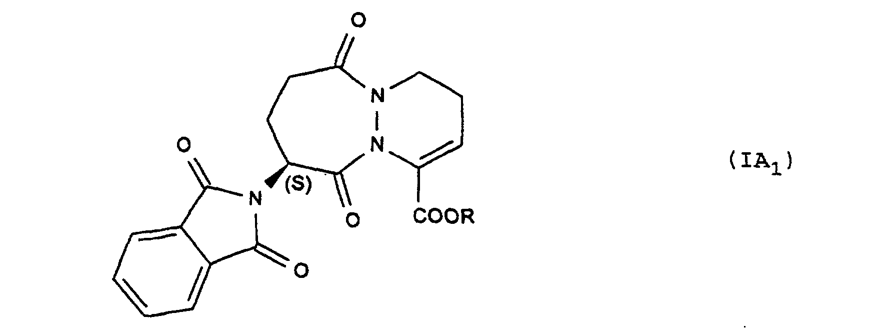 dans lesquels R représente un radical alkyle renfermant jusqu'à 8 atomes de carbone.

L'invention a tout spécialement pour objet les composés de formule (IA₁) dans lesquels R représente un radical 1,1-diméthyl éthyle.

L'invention a tout particulièrement pour objet le composé de formule (IA₁) dont la préparation est donnée ci-après dans la partie expérimentale.

L'invention a également pour objet un procédé de préparation caractérisé en ce que l'on soumet un composé de formule (II) sous la forme racémique au niveau du cycle à 6, à l'action d'un agent de déshydrogénation : 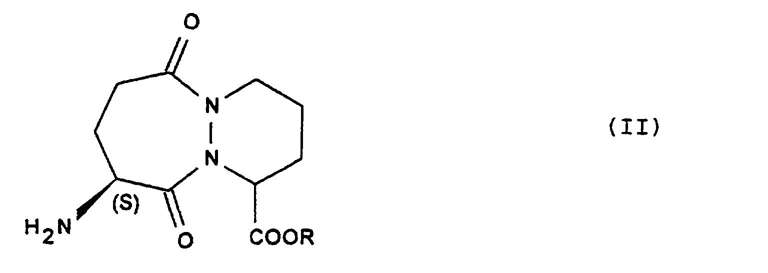 dans lequel R conserve sa signification précédente, la fonction amine pouvant être protégée pour obtenir le composé de formule (I) correspondant.

L'invention a plus particulièrement pour objet un procédé caractérisé en ce que le composé de formule (II) répond à la formule (IIA) : 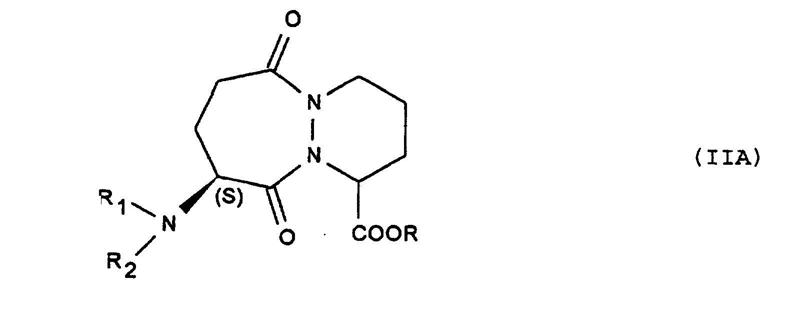 dans laquelle R₁, R₂ et R conservent leur signification précédente.

L'invention a également pour objet un procédé de préparation caractérisé en ce que la déshydrogénation est réalisée par l'utilisation d'une base forte d'un dérivé du soufre ou du sélénium puis d'un oxydant.

Les composés de formule (II) racémiques (SR+SS) au niveau du cycle à 6 chaînons, utilisés comme produit de départ du procédé de l'invention, sont des produits nouveaux et sont donc un objet de la présente invention. Ils peuvent être préparés comme indiqué ci-après dans la partie expérimentale.

Le produit racémique utilisé comme produit de départ de l'exemple 1, peut être préparé comme indiqué dans la préparation 1. Cette préparation peut être schématisée de la façon suivante :

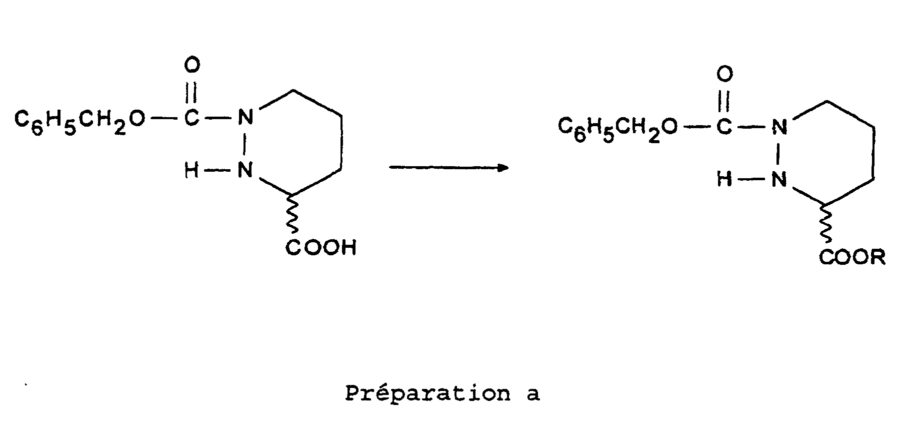

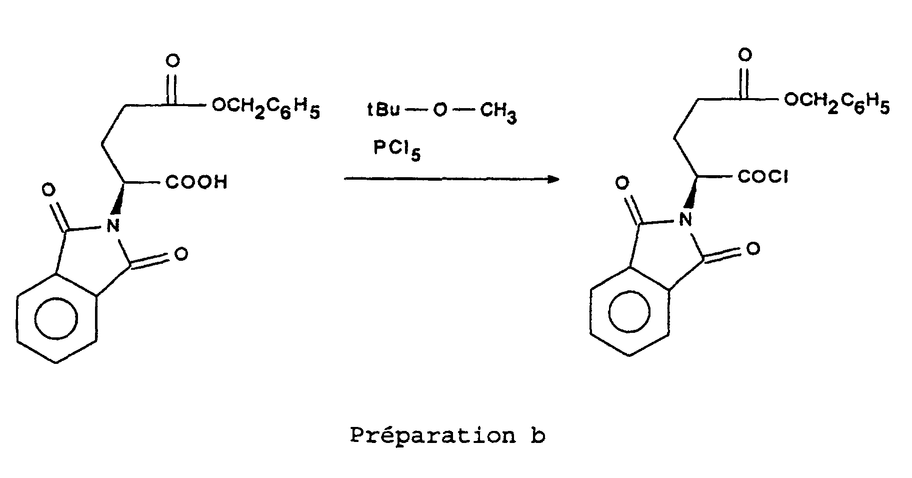

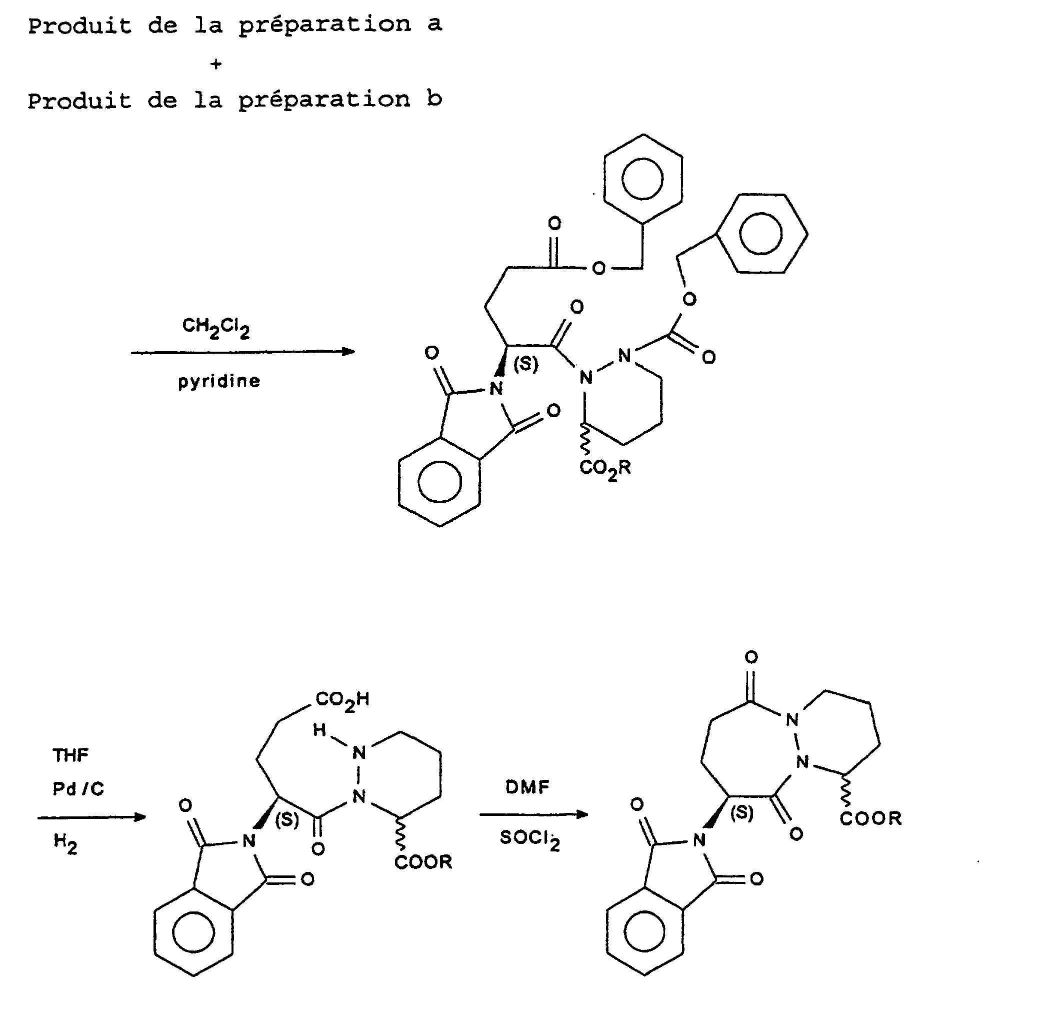

### Produit de départ de l'exemple 1

Les produits de départ utilisés pour cette préparation sont décrits ou peuvent être préparés comme indiqués dans J. Chem. Soc. Perkin Trans. 1 (1979) Vol. 6, p. 1451-1454 ou encore J. Chem. Soc. Chem. Comm. (1977) p. 635-636.

L'invention a également pour objet l'application des composés de formule (I), caractérisé en ce que l'on soumet un composé de formule (I) à l'action d'un agent de réduction pour obtenir les composés de formule (III) : 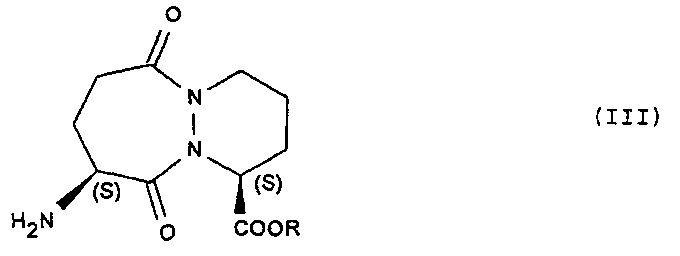 dans laquelle R conserve sa signification précédente et l'amine peut être libre ou protégée.

L'invention a notamment pour objet l'application des composés de formule (IA) à la préparation des produits de formule (IIIA) : 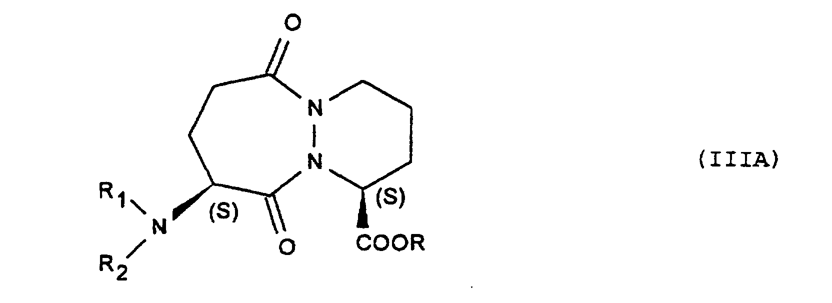 dans laquelle R, R₁ et R₂ conservent leur signification précédente.

La réaction de réduction peut être réalisée en utilisant par exemple l'hydrogène en présence de nickel de Raney, de palladium sur charbon, de dihydroxyde de palladium en présence de talc, de rhodium en présence d'alumine, de rhuthénium sur charbon. L'invention a plus particulièrement pour objet l'application caractérisée en ce que l'agent de réduction est l'hydrogène en présence de nickel de Raney.

Comme solvant, on peut utiliser l'acide acétique, le méthanol, l'éthanol, l'isopropanol, le diméthoxyéthane, la butanone, le DMF ou l'acétonitrile.

Les produits de formule (IIIA) sont des produits qui peuvent être utilisés pour préparer des produits doués de propriétés pharmacologiques intéressantes.

Le produit (IIIA) préparé dans la partie expérimentale est un produit connu, sa préparation et son utilisation dans la préparation de produits doués de propriétés pharmacologiques sont décrits par exemple dans EP 94095 ou encore dans J. Chem. Soc. Perkin Trans. 1, (1986) p. 1011 et suivantes.

Les autres produits de formule (III) peuvent être utilisés de façon analogue pour préparer les mêmes produits que ceux décrits dans le brevet et la publication cités ci-dessus.

Les produits de formules (II) et (IIA) de configuration SR ou sous forme d'un mélange (SR + SS) notamment le produit dont la préparation est donnée dans la partie expérimentale sont en eux-mêmes un objet de la présente invention.

Le produit de formule (IIIA) dans lequel R est un radical terbutyle et l'amine est protégée sous forme de phtalimido est décrit par exemple dans EP 94095.

Les exemples suivants illustrent l'invention.

### PREPARATION 1 : 9-(1,3-dihydro-1,3-dioxo-2H-isoindol-2-yl) octahydro-6,10-dioxo-6H-pyridazino[1,2-a] [1,2-diazépine-1-carboxylate de 1,1-diméthyl éthyle

### Préparation a : hexahydro 3(2H)-pyridazinedicarboxylate de 1-(phénylméthyle et 3-(1,1-diméthyl éthyle) J. Chem. Soc. Chem. Comm. (1977) p. 635-636 ou J. Chem. Soc. Perkin Trans. (1979) Vol. 6 p. 1451-1454

On introduit à 20°C, 920 µl de BF₃-Et₂O et une solution renfermant 18,85 g de trichloroacétimidate de terbutyle, 45,5 ml de cyclohexane et 57,5 ml de dichlorométhane dans une suspension renfermant 11,50 g de hexahydro-1,3(2H) pyridazinédicarboxyle de 1,1-phénylméthyle et 115 ml de dichlorométhane. Après traitement d'isolation et purification, on obtient le produit attendu.

### Préparation b : γ-(chlorocarbonyl)-1,3-dioxo-1H-isoindole-2(3H)-butanoate de phénylméthyle

On introduit sous atmosphère d'azote, 30 g de γ-carboxy-1,3-dioxo-1H-isoindol-(3H) butanoate de phénylméthyle (EP 94095), dans 81 ml de terbutylméthyléther. On refroidit à 0°-2°C et ajoute 17 g de pentachlorure de phosphore. On laisse revenir à la température ambiante, maintient 6 heures sous agitation, concentre sous pression réduite, porte à 41°C l'extrait sec obtenu, entraîne au toluène. On maintient le produit obtenu à la température ambiante et sous atmosphère d'azote. On le dilue pour utilisation dans le CH₂Cl₂, pour obtenir une solution 0,5 M.

### Stade A : 2-[1,5-dioxo-2-(1,3-dioxo-14-isoindol-2(3H)-yl)5-(phénylméthoxy)-pentyl] tétrahydro 1,3-(2H)-pyridazinedicarboxylate de 1-(phénylméthyle) 3-(1,1-diméthyléthyle)

On refroidit à 0°/-1°C une solution 0,5 M de 52 ml de produit de la préparation b dans le chlorure de méthylène. On ajoute 5,6 g de produit de la préparation a et 22 ml de dichlorométhane. On agite 3 heures à 0°+1°C et ajoute 1,77 ml de pyridine et 11 ml de chlorure de méthylène. On chasse le dichlorométhane sous pression réduite à 30°C, reprend à l'acétate d'éthyle, lave avec des solutions aqueuses de chlorure de sodium, de carbonate acide de sodium. On extrait à l'acétate d'éthyle, sèche, rince et extrait sous pression réduite. On chromatographie le produit obtenu sur silice en éluant avec le mélange heptane-acétate d'éthyle 60-40. On obtient 4,697 g de produit recherché. F ≤ 35°C.

### Stade B : Acide γ- [ [3- [ (1, 1-diméthyléthoxy) carbonyl] tétrahydro-2(1H)-pyridazinyl]carbonyl]-1,3-dioxo 1H-isoindole-2(3H)-butanoïque

On mélange à 20°C un mélange renfermant 4,61 g de produit du stade A, 47 ml de tétrahydrofuranne. On hydrogène à la température ambiante, en utilisant comme catalyseur 400 mg de palladium sur charbon. La réaction terminée, on filtre et rince au THF. On obtient 2,76 g de produit recherché.

### Stade C : 9-(1,3-dihydro-1,3-dioxo-2H-isoindol-2-yl) octahydro-6,10-dioxo-6H-pyridazino[1,2-a] [1,2-diazépine-1-carboxylate de 1,1-diméthyléthyle

On introduit à 0°/+2°C, une solution de 0,846 ml de chlorure de thionyle dans 2,6 ml de dichlorométhane dans un mélange renfermant 2,6 g de produit du stade B, 26 ml de chlorure de méthylène, 50 µl de diméthylformamide. On laisse revenir à la température ambiante. On maintient sous agitation pendant 6 h 20. On effectue les opérations d'isolation et purification et obtient le produit recherché.

### EXEMPLE 1 : (9S),9-(1,3-dihydro-1,3-dioxo-2H-isoindol-2-yl)-3,4,7,8, 9,10-hexahydro-6,10-dioxo-6H-pyridazino[1,2-a] [1,2]-diazépine-1-carboxylate de 1,1-diméthyléthyle

### a) Préparation de LDA

On ajoute vers -60°C en 10 minutes, sous azote et agitation 7,2 ml de butyllithium dans un mélange renfermant 20 ml de THF et 3,2 ml de diisopropylamine. On laisse la température remonter à 0°C, maintient 1 heure à 0°C et ramène à -60°C.

### b) Préparation du C₆H₅SeBr

On ajoute 0,26 ml de brome, à 10°C dans une solution renfermant 1,88 g de diphényldisélénium et 6 ml de THF. On maintient le mélange réactionnel sous agitation pendant 1 heure à 20°C.

### c) Réaction

On refroidit à -65°C un mélange de 3,44 g de 9-(1,3-dihydro-1,3-dioxo-2H-isoindol-2-yl) octahydro-6,10-dioxo-6H-pyridazino[1,2-a] 1,2-diazépine-1-carboxylate de 1,1-diméthyléthyle. On ajoute la solution de LDA préparée ci-dessus. On agite pendant 10 minutes et ajoute à une température de 50°C±5°C, la solution de C₆H₅SeBr. On laisse la température remonter vers 0°C et ajoute 4 ml d'eau, 1,2 ml d'acide acétique, 4 ml d'eau oxygénée. On laisse la température remonter à 10°C. On maintient le mélange réactionnel sous agitation pendant 1 heure et ajoute 40 ml d'une solution aqueuse de chlorure de sodium à 10 % et 80 ml d'acétate l'éthyle. On décante, lave avec la solution saturée en chlorure de sodium, et/ou bicarbonate de soude à 10 %. On évapore sous pression réduite. On chromatographie sur silice en éluant avec le mélange chlorure de méthylène-éther isopropylique. On isole 1,3 g de produit recherché.
α_{D} = + 126,5 - C = 0,335/MeOH

### APPLICATION : (1S,9S)-9-(1,3-dihydro-1,3-dioxo-2H-isoindol-2-yl)octahydro-6,10-dioxo-6H-pyridazino[1,2-a] [1,2]-diazépine-1-carboxylate de 1,1-diméthyléthyle

### [(1S-cis) 9-(1,3-dihydro-1,3-dioxo-2H-isoindol-2-yl)octahydro-6,10-dioxo-6H-pyridazino[1,2-a] [1,2]-diazépine-1-carboxylate de 1,1-diméthyléthyle.]

### a) Préparation du catalyseur

On agite pendant 2 heures à 60°C un mélange de 0,106 g de nickel de Raney (Jansen) et 2 ml d'hydroxyde de sodium. On lave le nickel à l'eau, ajoute une goutte d'acide acétique à la dernière eau de lavage. On lave ensuite le nickel à l'éthanol et l'acétate d'éthyle. On conserve le nickel obtenu sous pression réduite.

### b) Réduction

On ajoute 3 ml d'acétate d'éthyle à 0,053 g de nickel préparé comme précédemment. On ajoute à la suspension obtenue 0,0485 g du produit de l'exemple 1. L'étape d'hydrogénation a lieu à la température ambiante, jusqu'à ce qu'il n'y ait plus d'absorption d'hydrogène. Il y a absorption de 2 ml d'hydrogène.
CCM rf = 0,27 éluant éther isopropylique/chlorure de méthylène (50-50).

## Revendications

1. A titre de produits chimiques nouveaux, les composés de formule (I) : 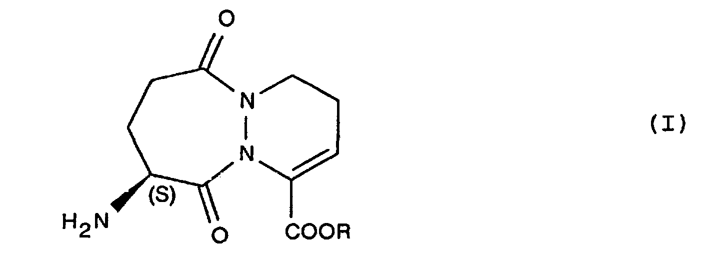 dans lesquels R représente un atome d'hydrogène, un radical alkyle ou aralkyle renfermant jusqu'à 18 atomes de carbone, la fonction amine pouvant être libre ou protégée.

2. A titre de produits chimiques nouveaux, les composés de formule (I) dans lesquels la fonction amine est protégée, qui répondent à la formule (IA) : 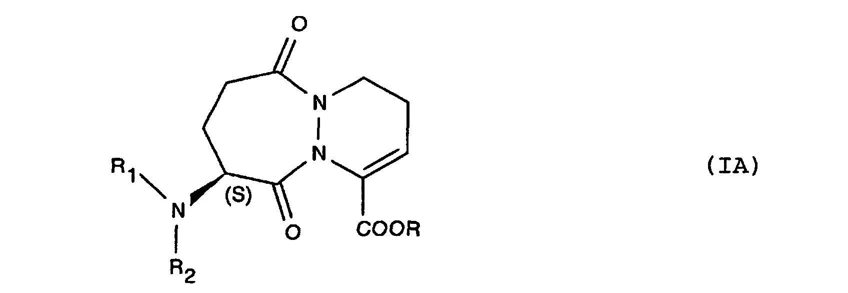 dans laquelle R est tel que défini à la revendication 1 et/ou bien R₁ représente un radical : 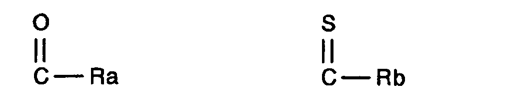 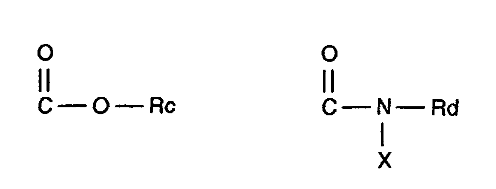
Ra, Rb, Rc et Rd représentant un radical alkyle ou aryle renfermant jusqu'à 18 atomes de carbone, ou un radical mono ou polycyclique renfermant un ou plusieurs hétéroatomes,
X représentant un atome d'hydrogène, un radical alkyle renfermant jusqu'à 8 atomes de carbone ou un radical aryle renfermant jusqu'à 14 atomes de carbone,
et R₂ représente un atome d'hydrogène,
ou bien R₁ et R₂ forment ensemble un radical mono ou polycyclique renfermant un ou plusieurs hétéroatomes.

3. A titre de produits chimiques nouveaux, les composés de formule (I) définis à la revendication 1 ou 2 dans lesquels R₁ et R₂ forment ensemble un radical polycyclique renfermant un ou plusieurs hétéroatomes.

4. A titre de produits chimiques nouveaux, les composés de formule (I) définis à la revendication 3, répondant à la formule (IA₁) : 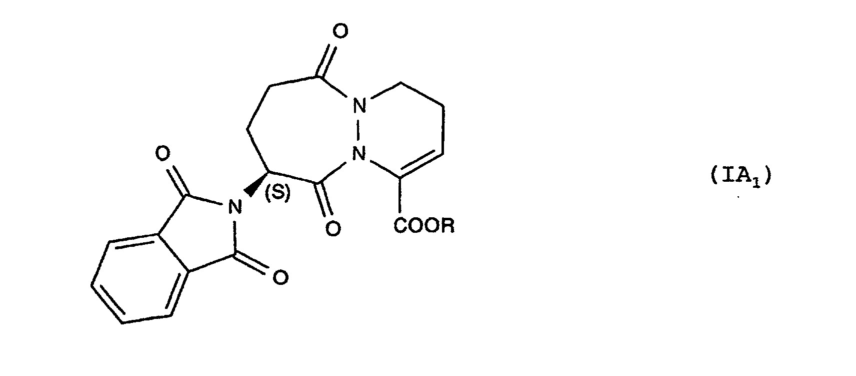 dans lesquels R représente un radical alkyle renfermant jusqu'à 8 atomes de carbone.

5. A titre de produits chimiques nouveaux les composés de formule (I) définis à l'une quelconque des revendications 1 à 4, dans lesquels R représente un radical 1,1-diméthyl éthyle.

6. A titre de produit chimique nouveau, le composé de formule (IA₁) dont le nom suit :
- (9S),9-(1,3-dihydro-1,3-dioxo-2H-isoindol-2-yl)-3,4,7,8,9,10-hexahydro-6,10-dioxo-6H-pyridazino[1,2-a][1,2] diazépine-1-carboxylate de 1,1-diméthyléthyle.

7. Procédé de préparation des composés de formule (I) définis à l'une quelconque des revendications 1 à 6, **caractérisé en ce que** l'on soumet à l'action d'une base forte , d'un dérivé du soufre ou du sélénium puis d'un oxydant, un composé de formule (II) sous la forme racémique: 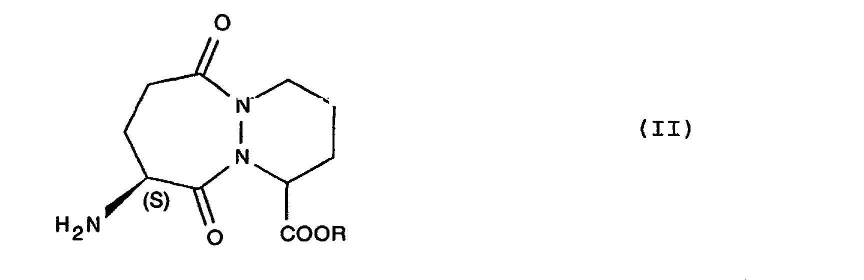 dans lequel R est tel que défini à la revendication 1, la fonction amine pouvant être protégée pour obtenir le composé de formule (I) correspondant.

8. Procédé de préparation selon la revendication 7, **caractérisé en ce que** le composé de formule (II) répond à la formule (IIA) : 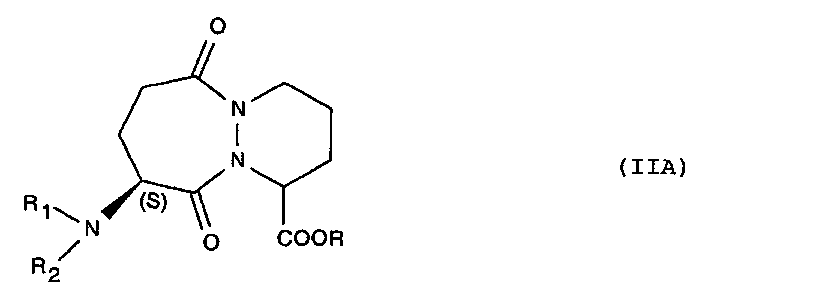 dans laquelle R₁, R₂ et R sont tels que définis à la revendication 2.

9. Application des composés de formule (I) définis à l'une quelconque des revendications 1 à 6, **caractérisé en ce que** l'on soumet un composé de formule (I) à l'action d'hydrogène en présence de Nickel de Raney pour obtenir le composé de formule (III) : 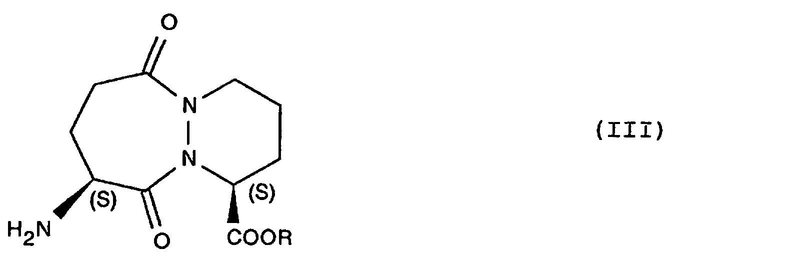 dans laquelle R tel que défini à la revendication 1 et l'amine peut être libre ou protégée.

10. Application selon la revendication 9 à la préparation des composés de formule (IIIA) : 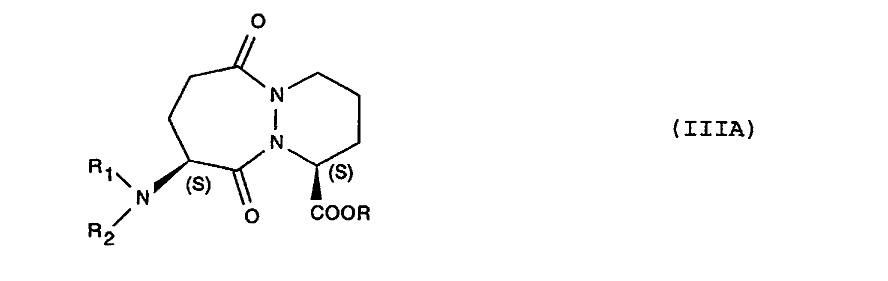 dans laquelle R, R₁ et R₂ sont tels que définis à la revendication 2.

## Patentansprüche

1. Neue chemische Produkte in Form von Verbindungen der Formel (I): 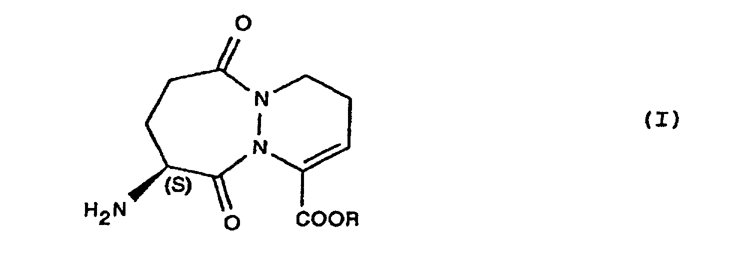 worin R für ein Wasserstoffatom oder einen Alkyloder Aralkylrest mit bis zu 18 Kohlenstoffatomen steht und die Aminfunktion frei oder geschützt sein kann.

2. Neue chemische Produkte in Form von Verbindungen der Formel (I) mit geschützter Aminfunktion der Formel (IA): 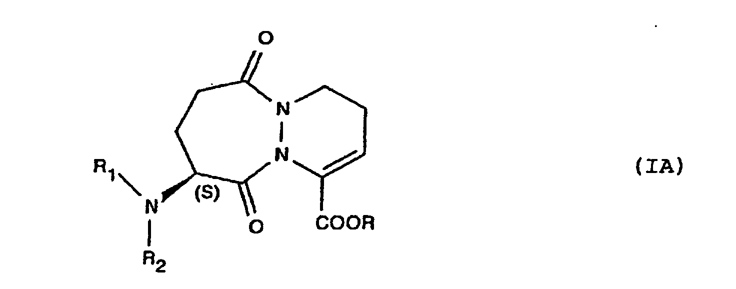 worin R die in Anspruch 1 angegebene Bedeutung besitzt und/oder R₁ für einen Rest: 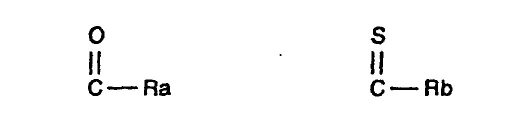 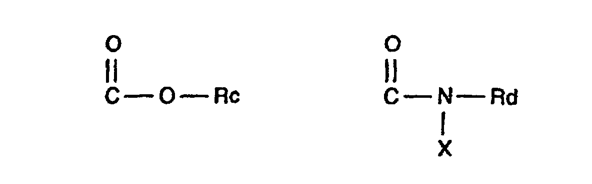 steht, wobei Ra, Rb, Rc und Rd für einen Alkyloder Arylrest mit bis zu 18 Kohlenstoffatomen oder einen mono- oder polycyclischen Rest mit einem oder mehreren Heteroatomen stehen,
X für ein Wasserstoffatom, einen Alkylrest mit bis zu 8 Kohlenstoffatomen oder einen Arylrest mit bis zu 14 Kohlenstoffatomen steht
und R₂ für ein Wasserstoffatom steht
oder R₁ und R₂ gemeinsam einen mono- oder polycyclischen Rest mit einem oder mehreren Heteroatomen bilden.

3. Neue chemische Produkte in Form von Verbindungen der Formel (I) nach Anspruch 1 oder 2, worin R₁ und R₂ gemeinsam einen polycyclischen Rest mit einem oder mehreren Heteroatomen bilden.

4. Neue chemische Produkte in Form von Verbindungen der Formel (I) nach Anspruch 3 der Formel (IA₁): 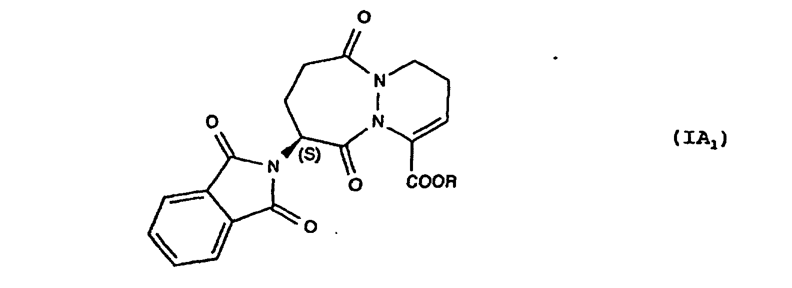 worin R für einen Alkylrest mit bis zu 8 Kohlenstoffatomen steht.

5. Neue chemische Produkte in Form von Verbindungen der Formel (I) nach einem der Ansprüche 1-4, worin R für einen 1,1-Dimethylethylrest steht.

6. Neues chemisches Produkt in Form der Verbindung der Formel (IA₁) mit dem folgenden Namen:
- (9S),9-(1,3-Dihydro-1,3-dioxo-2H-isoindol-2-yl)-3,4,7,8,9,10-hexahydro-6,10-dioxo-6H-pyridazino[1,2-a][1,2]diazepin-1-carbonsäure-1,1-dimethylethylester.

7. Verfahren zur Herstellung von Verbindungen der Formel (I) nach einem der Ansprüche 1-6, **dadurch gekennzeichnet, daß** man eine Verbindung der Formel (II) in racemischer Form: 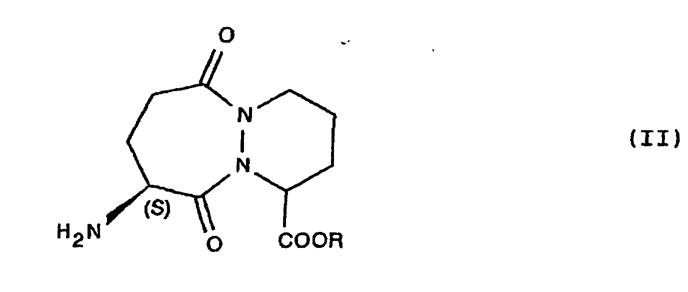 worin R die in Anspruch 1 angegebene Bedeutung besitzt und die Aminfunktion geschützt sein kann, mit einer starken Base, einem Schwefel- oder Selenderivat und dann mit einem Oxidationsmittel behandelt, wobei man die entsprechende Verbindung der Formel (I) erhält.

8. Herstellungsverfahren nach Anspruch 7, **dadurch gekennzeichnet, daß** die Verbindung der Formel (II) der Formel (IIA) entspricht: 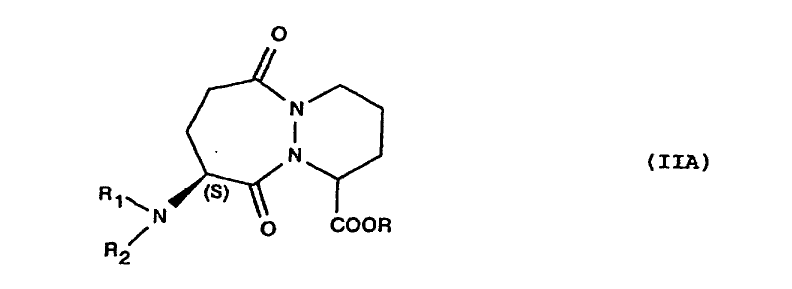 worin R₁, R₂ und R die in Anspruch 2 angegebene Bedeutung besitzen.

9. Verwendung der Verbindungen der Formel (I) nach einem der Ansprüche 1 bis 6, bei der man eine Verbindung der Formel (I) in Gegenwart von Raney-Nickel mit Wasserstoff behandelt, wobei man die Verbindung der Formel (III): 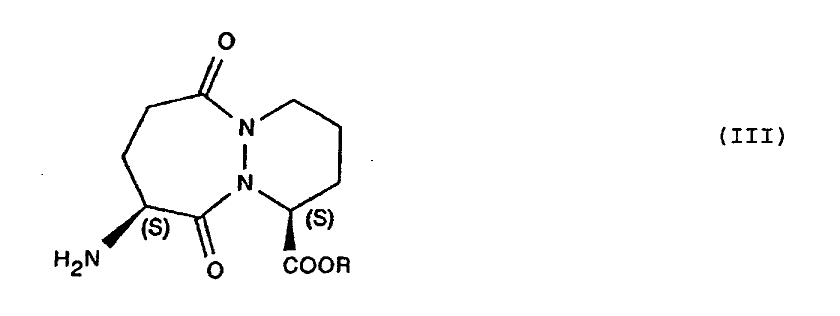 worin R die in Anspruch 1 angegebene Bedeutung besitzt und das Amin frei oder geschützt sein kann, erhält.

10. Verwendung nach Anspruch 9 zur Herstellung der Verbindungen der Formel (IIIA): 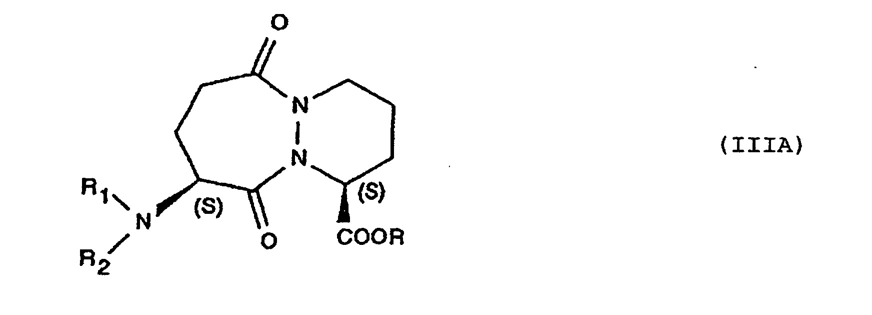 worin R₁, R₂ und R die in Anspruch 2 angegebene Bedeutung besitzen.

## Claims

1. As novel chemical products, the compounds of formula (I): 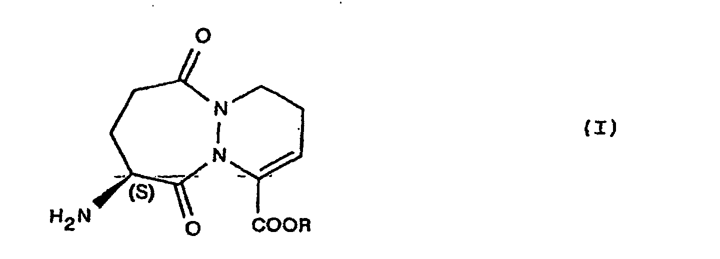 in which R represents a hydrogen atom, an alkyl or aralkyl radical containing up to 18 carbon atoms, it being possible for the amine functional group to be free or protected.

2. As novel chemical products, the compounds of formula (I) in which the amine functional group is protected, which correspond to formula (IA): 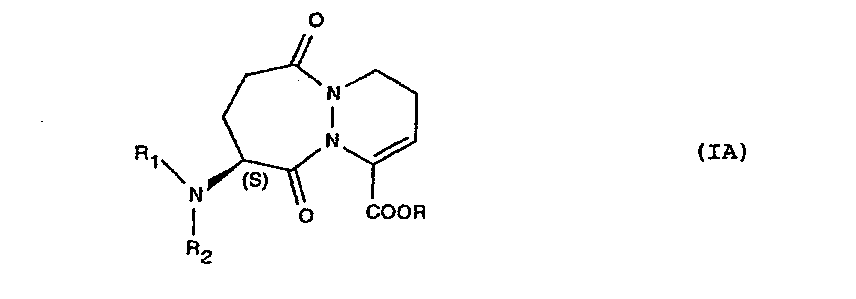 in which R is as defined in Claim 1 and/or R₁ represents a radical: 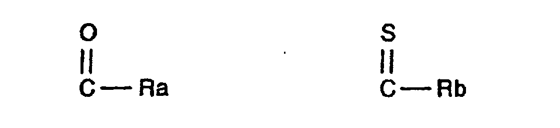 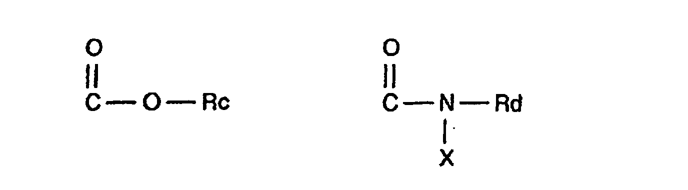 Ra, Rb, Rc and Rd representing an alkyl or aryl radical containing up to 18 carbon atoms, or a mono- or polycyclic radical containing one or more heteroatoms,
X representing a hydrogen atom, an alkyl radical containing up to 8 carbon atoms or an aryl radical containing up to 14 carbon atoms,
and R₂ represents a hydrogen atom,
or alternatively R₁ and R₂ form together a mono- or polycyclic radical containing one or more heteroatoms.

3. As novel chemical products, the compounds of formula (I) defined in Claim 1 or 2 in which R₁ and R₂ form together a polycyclic radical containing one or more heteroatoms.

4. As novel chemical products, the compounds of formula (I) defined in Claim 3, corresponding to formula (IA₁): 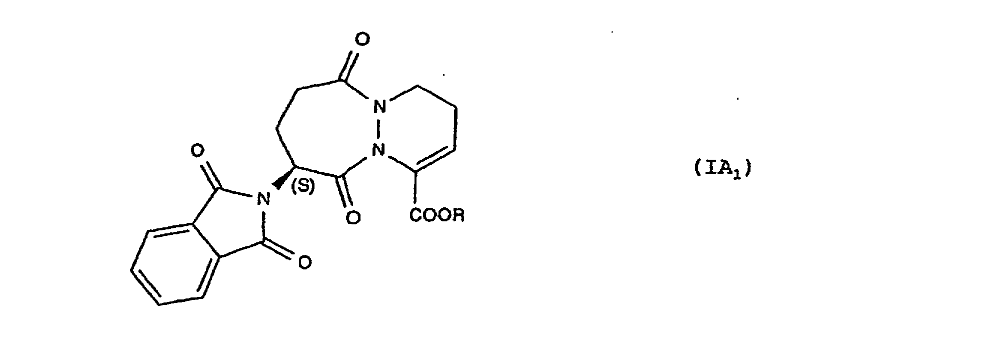 in which R represents an alkyl radical containing up to 8 carbon atoms.

5. As novel chemical products, the compounds of formula (I) defined in any one of Claims 1 to 4, in which R represents a 1,1-dimethylethyl radical.

6. As novel chemical product, the compound of formula (IA₁) whose name follows:
- 1,1-dimethylethyl (9S), 9-(1,3-dihydro-1,3-dioxo-2H-isoindol-2-yl)-3,4,7,8,9,10-hexahydro-6,10-dioxo-6H-pyridazino[1,2-a][1,2]diazepine-1-carboxylate.

7. Method for preparing the compounds of formula (I) defined in any one of Claims 1 to 6, **characterized in that** there is subjected to the action of a strong base, of a derivative of sulphur or of selenium and then of an oxidizing agent, a compound of formula (II) in racemic form: 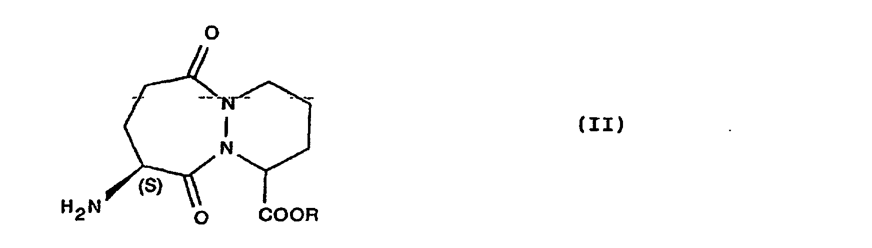 in which R is as defined in Claim 1, it being possible for the amine functional group to be protected so as to obtain the corresponding compound of formula (I).

8. Method of preparation according to Claim 7, **characterized in that** the compound of formula (II) corresponds to formula (IIA): 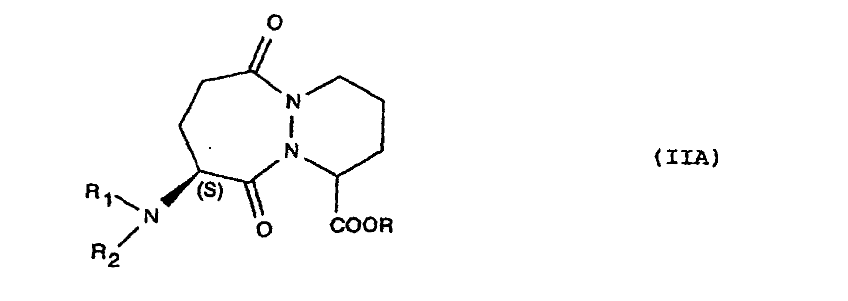 in which R₁, R₂ and R are as defined in Claim 2.

9. Application of the compounds of formula (I) defined in any one of Claims 1 to 6, **characterized in that** a compound of formula (I) is subjected to the action of hydrogen in the presence of Raney nickel so as to obtain the compound of formula (III): 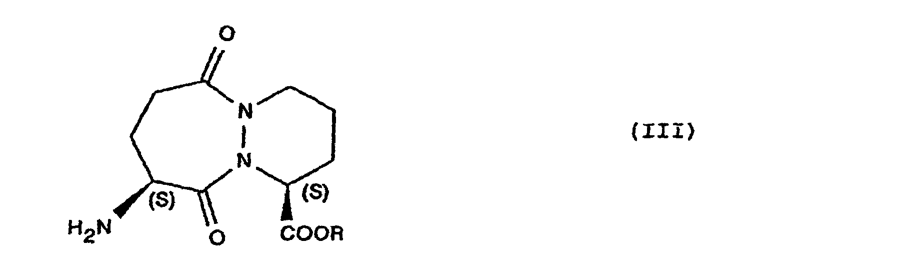 in which R is as defined in Claim 1 and the amine may be free or protected.

10. Application according to Claim 9 to the preparation of the compounds of formula (IIIA): 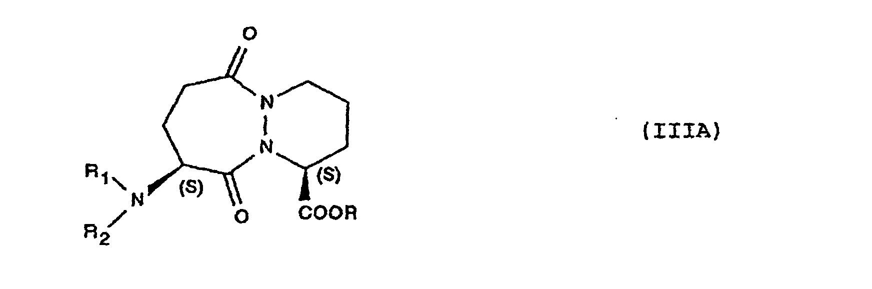 in which R, R₁ and R₂ are as defined in Claim 2.
